# EUROPEAN PATENT SPECIFICATION

(11) **EP 2 400 937 B1**
(45) Date of publication and mention of the grant of the patent: **06.11.2013**
(21) Application number: 10726653.8
(22) Date of filing: 11.02.2010
(51) Int. Cl.: A61F 5/451, A61G 5/10, A61G 7/02

(54) **SUPPORT UNIT FOR PHYSICALLY HANDICAPPED PATIENTS**
HILFEEINHEIT FÜR KÖRPERLICH BEHINDERTE PATIENTEN
UNITÉ D'ASSISTANCE POUR HANDICAPÉS PHYSIQUES

(30) Priority: 26.02.2009 TR 200901453
(43) Date of publication of application: 04.01.2012
(73) Proprietor: Pirli, Aslan Ali, Kocaeli (TR)
(72) Inventor: Pirli, Aslan Ali, Kocaeli (TR)
(74) Representative: Beetz & Partner
(86) International application number: PCT/TR2010/000032
(87) International publication number: WO 2010/098731

(56) References cited:
- EP-A1- 1 219 270
- WO-A2-2008/041959
- US-A- 5 058 222

## Description

The invention is about a support unit for physically handicapped patients which is used for conscious or unconscious physically handicapped patients, provides the patient's needs through automatic or manual control, is turned into a bed or a wheeled chair for physically handicapped patients, ensures that the patient performs physical acts, enables blood circulation by massaging to the feet's lower base through the vibration of the step under the patient's feet, ensures that the patient defecates and the dejection is transferred to the sewage system wholly automatically without human touch hygienically depending on the equipment under the seat section (22) of the chair and provides the patient with free scope. Such a support unit has been disclosed in WO-A-2008/041959.

Man kinds have not fulfilled their vital needs, like defecation, by themselves once or few times all their life because of the incapability of physical movement. It is very demanding to be helped and cleaned, because of the inadequate movement ability, by the family members or nursemaids. These kinds of people generally attached to one room cause their relatives to suffer pain and the people having this kind of patients have great troubles. That situation results in a decrease in those people's life quality. In the same way, the reality that the patients turn into trouble for their relatives is a source of sorrow for the patients themselves which causes psychological issues for them.

The support unit for physically handicapped patients saves the unconscious patients and the physically handicapped patients, who are the most serious cases in need of care, from being wholly depended on another person. The invention is ideal for using in fulfilling the needs of the elder, the bedridden, the handicapped and the paralyzed who are deprived of the movements that will fulfill their normal needs and who are in need of nursing.

### THE EXPLANATION OF THE INVENTION:

The invention consists of those sections: the multi-functional underpants (17) which is fastened on the patient's body by dividing the gluteus and centering the anus without touching upon the periphery of anus, has electronic and electro mechanic equipment, is multi-layered and does the hygienic cleaning of the patient, the waste reserve storage (19) which, when fastened on the patient, removes the waste as soon as the waste leaves the patient's body, in an entirely controlled way without contaminating the patient's body and the surroundings, the waste transfer station (30) which can transfer the waste, to the sewerage system, automatically by moving with the help of sensors in the body of the unit as a result of fullness of the waste reserve storage (19).

### Multi-Functional Underpants:

It is designed for unconscious physically handicapped patients. It is an underpants which is attached to the unit through equipment existent in it in order to ensure that the patient fulfill his/her detection need and functions like hygienic cleaning without problem and does not limit the movements of the patient by the support of the unit equipments.

The inner surface level (1) of the part of the multi-functional underpants (17) that touches the patient's body is of antibacterial quality and is covered with flexible material and cloth that does not affect blood circle. The multi-functional pant (17) is designed as to divide the gluteus and grab the patient's body ergonomically. It is worn and fixed to the patient by being arranged for every size through demounted additions.

The multi-functional pant (17) is consisted of two levels. In the middle of the first inner surface level (1) which touches the patient body, there is a proper-sized hole (2) which is suitable for the middle of the anus and surrounds the anus. That hole, at the same time, centers waste removal pump (5) and, being attached to that, the strait (7) part which is a waste entry part to the anus, and supports them. There are cabins which are divided into levels (6) that constitute proper distance between the outer surface of the multi-functional pant's first inner level (1) that touches the body and the inner surface (4) which is fixed to the second outer level (3). The aim of that distance is to constitute cabin for protecting the electromechanically equipped waste removal pump (5) from the pressure that the patient makes on the pant. The waste entry strait (7) of the mechanically equipped waste removal pump (5) is steered through the hole (2) which is in the middle of the multi-functional pant's (17) first inner level (1) that touches the patient's body and which is suitable for the middle of the anus.

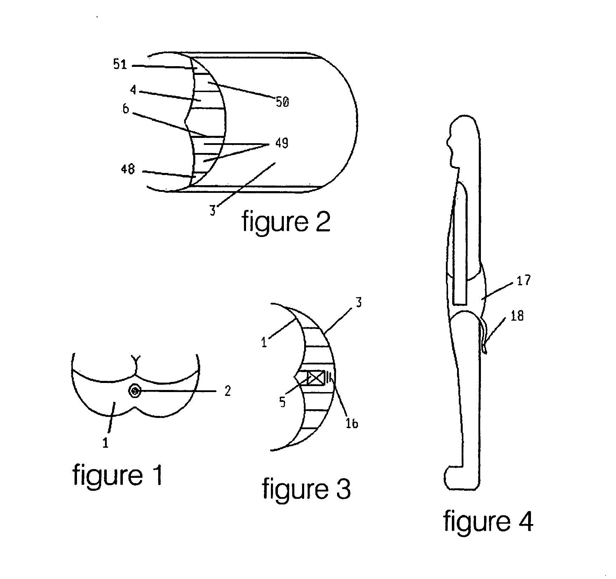

The waste entry strait part (7) of the electro mechanic waste removal pump (5) ensures that the waste which comes out of the anus is transferred to the pump without contaminating the patient and his/her surroundings and through that way, the hygienic cleaning of the anus is performed. That waste entry strait part (7), which surrounds the anus, is close to the anus but does not touch the anus, and belongs to the waste removal pump (5), is of cylindrical shape in the width of the anus' opening diameter. The edges of the point (8), which belongs to the waste entry strait part (7) of the waste removal pump (5) and touches the anus' surroundings, are flexible and thin. That point is made from silicone or similar hygienic flexible material that does not hurt or irritate anus's surroundings during the touch on the edges of the anus (as shown in the figures 1, 2, 3 and 5).

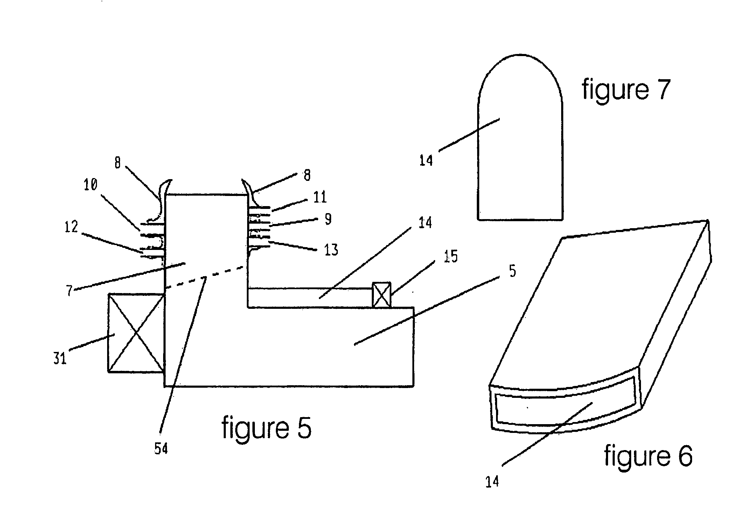

There are a micro camera fixation channel (9), a waste control sensor fixation channel (10), an air entry fixation channel (11), a water entry fixation channel (12), a light source, which enlightens the surroundings of the anus, fixation channel (13) on the outer edge of electromechanical waste removal pump's (5) waste entry strait part (7) (Figure 5). The equipments that will be mounted on those fixation channels are alerted by the sensor as soon as the wastes come out of the patient's body. Electromechanically equipped pump engine (31) operates after the latch (14), which closes the gap between, in the multi-functional pant (17), the waste entry strait part (7) of the electro mechanic waste removal pump (5) and the hole (2) part surrounding the anus, is unbolted by the electro mechanism (15) that activates the latch. The latch (14), after the defecation finishes, by closing the waste entry strait part (7) between anus and the pump, cuts the connection between the waste removal pump (5), the waste storage in the unit and anus. However, electromechanically equipped waste removal pump (5) has self-cleaning equipment through sprinkler water system. Thanks to the equipment, it is ensured that the surroundings of the anus remain hygienic by the cut of the operation between the wastes that may be remained in the pump and the anus and also the cut of the smell transfer. Thus, the latch (14) is an apparatus that is designed with the aim of providing the anus with hygienic atmosphere. The latch (14) or figures of design is opened or closed through mechanisms like valves. When the wastes leave the body, the pneumatic diaphragm (16), which swells with the current air under the waste removal pump (5), through swell mechanic movement or electro mechanism, by raising the waste removal pump (5) up towards the anus and ensuring that the point (8) of the pump's upper waste entry strait part (7), that touches the surroundings of the anus, touches the surroundings of the anus (2) with proper pressure, hinders the leak of the wastes outside. Except those practices, if the patient is in rest position, the pneumatic diaphragm (16) that swells with the current air under the waste removal pump (5), by raising the waste removal pump up towards the anus with the swell mechanic movement or electro mechanism, by perpetuating the touch of the anus on its surroundings is for supporting the entry of the leaks like liquid that may come from anus into the waste removal pump (5). That precaution is designed in order to hinder the wastes from contaminating the body. When there is a change in the position of the patient, meaning the upright or sitting position, the pneumatic diaphragm (16) that swells with the current air under the waste removal pump (5), with the swell mechanic movement or electro mechanism, by replacing the waste removal pump (5), ensures that the connection of the pump's point (8) that touches on the surroundings of the anus with the body is cut.

Whatever position the patient is in, rest, upright, sitting, lying on back or lying on both sides, the multi-functional pant (17) with the help of its technical equipment and unit, fulfills the patient's defecation need automatically and does not limit the movements of the patient. The multi-functional pant (17) transfers the wastes to the waste reserve storage (19) through a pipe (18). At the same time, because the electromechanically equipped waste removal pump (5) is also on, solid wastes are transferred to the waste reserve storage (19) which is available in the unit.

### Mobile Multi-Functional Pant:

The mobile multi-functional pant (17) is designed for astronauts, little children and physically handicapped traveling patients etc. It is a mobile multi-functional pant (17) which is designed for fulfilling the needs of those people who travel for various reasons and which has an electronic equipment to practice the necessary instructions that are for the electro mechanic mechanisms on it to fulfill the procedures. It performs an entirely hygienic cleaning and it transfers the wastes, without contaminating them on the person's body, to the current storage in it. The mobile multi-functional pant (17) does not limit the movements of the people wearing it.

The inner surface level (1) of mobile multi-functional pant (17)'s part that touches the body of the patient is covered with flexible material and cloth which do not make the patient sweat, affect the blood circle and are of antibacterial quality. The multi-functional pant (17) is designed as to divide the gluteus and grab the patient's body ergonomically. It is worn and fixed to the patient by being arranged for every size through demounted additions.

The mobile multi-functional pant (17) is consisted of two levels. In the middle of the first inner level (1) which touches on the patient body, there is a proper-sized hole which is suitable for the middle of the anus and which surrounds the anus. That hole, at the same time, centers waste removal pump (5) and, being attached to that, the strait part (7) part which is a waste entry part to the anus and supports them. There are cabins which are divided into levels (6) that constitute proper distance between the outer surface of the mobile multi-functional pant's inner surface level (1) that touches on the body and the inner surface (4) which is fixed to the second outer level (3). The aim of that distance is to protect the electromechanically equipped waste removal pump (5) from the pressure that the patient makes on the pant and to constitute cabins for waste reserve storage (49), clean water storage (50), DC energy battery (51). The waste entry strait part (7) of the mechanically equipped waste removal pump (5) is steered through the hole which is in the middle of the mobile multi-functional pant's (17) first inner surface level (1) that touches on the patient's body and which is suitable for the middle of the anus. The waste entry strait part (7) of the waste removal pump (5) ensures that the waste which comes out of the anus is transferred to the pump without contaminating the patient and his/her surroundings and through that way, the hygienic cleaning of the anus is performed. That waste entry strait part (7), which surrounds the anus, is close to the anus but does not touch the anus, of the waste removal pump (5) is of cylindrical shape in the width of the anus' opening diameter. The edges of the point (8), which belongs to the waste entry strait part (7) of the waste removal pump (5) and touches the anus' surroundings are flexible and thin. That point is made from silicone or similar hygienic flexible material that does not hurt or irritate anus's surroundings during the touch on the edges of the anus (as shown in the figures 1, 2, 3 and 5).

There are a micro camera fixation channel (9), a waste control sensor fixation channel (10), an air entry fixation channel (11), a water entry fixation channel (12), a light source, which enlightens the surroundings of the anus, fixation channel (13) on the outer edge of electromechanical waste removal pump's waste entry strait part (7) (Figure 5). The current electronic control center (48) in the mobile multi-functional pant (17) is alerted by the sensor as soon as the waste comes out of the patient's body. The equipments that will be mounted on those fixation channels and the consistent electronic control center (48) in the mobile multi-functional pant (17) are alerted by the sensor as soon as the wastes come out of the patient's body. Upon the alert, the electronic control center (48) operates. After the latch (14) which closes the gap between, in the mobile multifunctional pant (17), the waste entry strait part (7) of the electro mechanic waste removal pump (5) and the hole (2) part surrounding the anus, is unbolted by the electro mechanism (15) that activates the latch, the electronically equipped pump engine (31) operates, the pneumatic diaphragm (16) through swell mechanic movement by raising the waste removal pump (5) up towards anus and by ensuring that the point (8) of the upper waste entry strait part (7), which touches the surroundings of the anus (2), touches the surroundings of the anus with a proper pressure hinders the leak of waste outside. However, electromechanically equipped waste removal pump (5) has self-cleaning equipment through sprinkler water system. It is ensured that the surroundings of the anus remain hygienic by the cut of the operation between the wastes that may be remained in the pump and the wastes that is in the waste reserve storage (49) of the multi-functional pant (17) and the anus and also the cut of the smell transfer. Thus, the latch (14) is an apparatus that is designed with the aim of providing the anus with hygienic atmosphere. The latch (14) or other figures of design is opened or closed through mechanisms like valves.

The electronically equipped waste removal pump (5) transfers the solid wastes to the current waste storage (49) of the mobile multi-functional pant (17). Then it performs the hygienic cleaning of the patient. The waste transfer mechanism (29) that is settled under the waste storage (49) of the mobile multi-functional pant (17) has technical qualities that are similar to the multi-functional pant's but here the waste reserve storage is the waste reserve storage of the mobile multi-functional pant. When the wastes are full of in the waste reserve storage (49) of the mobile multi-functional pant (17), the waste reserve storage gives caution warnings through the current level sensor in it.

It is ensured that the mobile multi-functional pant, like in the multi-functional pant, is attached to the unit in order to empty the wastes in the waste reserve storage (49) of the mobile multi-functional pant (17) and transfer the wastes to the waste transfer mechanism (29).

The mobile multi-functional pant (17) with the support of its current technical equipment automatically provides the needs of the patient. It transfers the wastes to the waste reserve storage (49), during those procedures, it does not contaminate the defecation wastes of the people anywhere but the opening of anus (2) and it does not limit the movements.

### Waste Transfer Mechanism:

Waste transfer mechanism (29) is settled in the unit and transfers the wastes in the waste reserve storage (19), where the wastes that are removed from the patient are collected, to the sewage system, through its mechanical equipment, by the directions of the unit, by attaching waste transfer station (30) automatically to the sewage system.

The waste transfer mechanism (29) takes place under the patient in the cabin of the unit (23). Waste transfer mechanism (29) has alignment channels (47) that enable the waste transfer mechanism to clamp to the waste transfer station (30). Those channels ensure that the waste transfer mechanism (29) is clamped and separated with the waste transfer station (30) by activating it through engine with a reducer (53) or pneumatic mechanic movements. Waste transfer mechanism (29) has a pipe (18) through which the multi-functional pant (17) attached on the patient removes the waste from the patient and has waste reserve storage (19) where the wastes are collected. The wastes are stored in the waste reserve storage (19). The gases of wastes that are stuck because of the wastes in the waste reserve storage (19) are filtered with gas filter (32) and through that way the smell is handled. There is a waste transfer pump (33), which is fixed to the storage, which mixes the wastes and transfers to the station, under the waste reserve storage (19). The waste reserve storage (19) is funnel-shaped and curved and has a very slippery surface in order to ensure that the wastes flow to the waste entry opening part of the waste transfer pump (33). Those qualities of the waste reserve storage (19) hinder the accumulation of the wastes on the edges and ensure that the waste transfer pump (33) flows to the waste entry opening. It alerts the unit through waste level sensor (34) when the waste reserve storage (19) is full of wastes. The unit notifies the warning of the waste reserve storage (19) through voice or light to the people outside the unit and it gives the warning for a direction to the waste transfer station (30). The waste transfer pump (33) in the waste transfer mechanism (29), until the directed unit automatically takes it to the waste transfer station (30) and stops in the line of clamping, by continuing operating, circulating the wastes in the inner waste circulation channel (35), transforming through the waste reserve storage (19), diluting the viscosity of the wastes that are subsided, providing the wastes with fluidity, simplifies the pumping of the wastes to the waste transfer station (30). The mechanism that operates the clamp that is fixed by passing before the waste transfer pipe (37) through which the waste transfer pump (33) pumps the wastes is a mechanism (36) which has one entry and two exits and through which the wastes, divided into two, pass. One (37) of the waste transfer pipes of the mechanism (36) operating the valve, which are divided into two, transfers the wastes to the station, the other (37) circulates the wastes by returning back to the inside of the storage. There is a waste redirecting clamp (38) in the point of the part where the pipes meet, which is controlled by the unit, has mechanical movements and will close the one of holes on the pipe on its both sides. The waste redirecting clamp (38) ensures that the wastes, which the waste transfer pump (33) transfers, circulates by the direction it gets from the unit by opening the entry of one of pipes and closing the entry of the other or ensures that the clamping occurs for its pumping to waste transfer station (30). If the clamping of the waste transfer mechanism (29), which is attached to the unit, to the waste transfer station (30) becomes unsuccessful, the procedure does not continue. If the clamping becomes successful, the unit gives direction to the waste transfer mechanism (29) to make the wastes transfer to the waste transfer station (30).

Waste transfer mechanism (29), by operating upon the direction it gets, from the waste transfer station (30) to the unit, with clean water pipe (39), transfers clean water, transfers energy to the station through DC energy transfer battery (40) and it performs automatically that the wastes are transferred to the waste transfer station (30). The waste transfer pump (33) current in waste transfer mechanism (29) with the help of sprinklers current in it, after the procedure of waste transfer, cleans itself through washing. After all those procedures finish, waste transfer mechanism (29) alerts the unit with the warning of the procedures' ending. All the procedures that waste transfer mechanism (29) performs are performed without human intervention automatically.

### Station of Waste Transfer to the Sewage System:

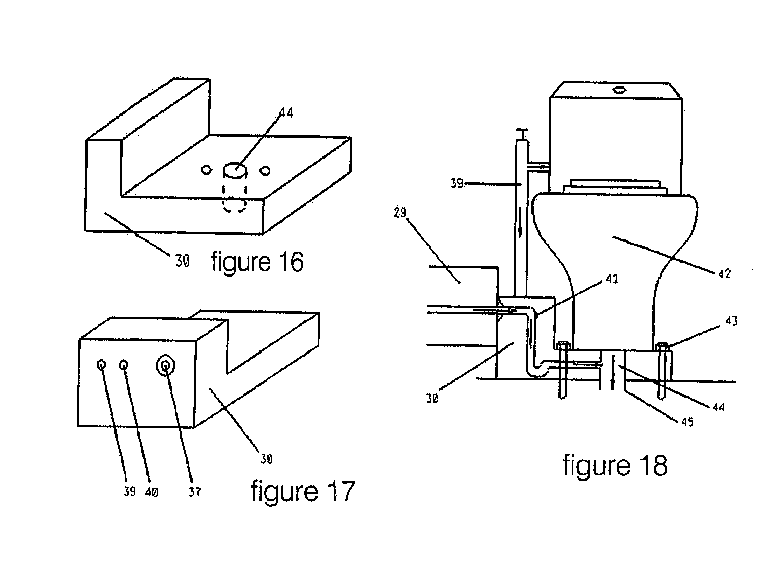

As shown in the figures 16 and 17, waste transfer station is settled under the closet. Waste transfer station (30) is designed for settling connection with the sewage system, clamping to the unit, and without any problems transferring the wastes transferred from the unit to the sewage system. Waste transfer station (30) has equipments that transfer clean water to the unit and clean themselves.

Waste transfer station (30) is an equipment that is out of the unit and combines the unit. There are two current solenoids in the waste transfer station (30). One of those solenoids is attached to the main water system through a clean water pipe (39). The other one is that transfers water to the sprinkler (41) which washes the parts of the waste transfer station through which wastes transfer to the sewage system. Those solenoids work through the energy transfer with DC energy battery (40) by the unit during the clamping of the unit with waste transfer station (30) and through the direction the unit gives. Waste transfer station (30), by the dismembering of the closet fixation screws (43) that fix the closet (42) that has a connection with the sewage system in the places where the patient takes place like toilet, bathroom etc, replaces the closet (42) and the waste pipe part of the station (44) replaces the sewage system pipe (45). Then the closet (42) is settled on the waste transfer station (30). The closet (42), with the screws (43) of enough length that are given with waste transfer station (30), is fixed on the ground with the waste transfer station. In addition to that, if there is not closet (42), the waste transfer station (30) has equipments that will be mounted on the places that are near to the sewage system etc. or that are attached to the sewage system. In order for the waste transfer station (30) to help the unit clamp by performing transferring the wastes that are transferred from the unit, there is an alignment panel (52) which is attached with the waste transfer station (30), is mounted on the ground and has two channels. The alignment channels (47) of the unit alignment panel (52) aligns the unit to the waste transfer station (30) in order to ensure that the two front wheels of the unit pass and make the unit clamp to the waste transfer station (30) without any problems. While, after the clamping, the alignment channels complete the transfer of the wastes, that have been transferred to the waste transfer station (30) from the unit, to the sewage system without contaminating the surroundings, the waste transfer station (30), upon the direction by the unit, by opening the clean water pipe (39) solenoid that is one of the solenoids consistent in the waste transfer station, transfers the clean water to the storage of the unit. After the procedure of the waste transfer pipe (37) is finished, by opening the sprinkler (41) solenoid finished the cleaning of the target parts of the station. Thus, the unit, by performing all these procedures automatically, leaves the unit.

### Squatting Position:

The support unit for conscious or unconscious physically handicapped patients characterized in that; it brings the patient to the squatting position while it fulfills the patient's defecation need and supports that the wastes are easily come out of the patient's body and the patient is relieved.

While the patient is fulfilling the defecation need, whatever position the patient is, as soon as the wastes leave the body, with the warning of the waste control sensor (10) current in the multi-functional pant (17), the unit brings the patient to the squatting (chair) position. Then the step (20) under the patient's feet and mobile step frame (28), to which the step is attached to, through the help of the mechanism by rising up brings the patient to the squatting position. That mechanic movement leads the patient's knees to make pressure on the abdominal region of the patient so it performs the cleaning of the intestines by helping the wastes transfer out of the body of the patient.

### Bed Position:

The support unit for conscious or unconscious physically handicapped patients characterized in that; it turns into a bed of standard bed size when wished and turns into a chair when wished. By these means, it becomes possible that the patient is wandered in various places with the unit.

When the patient is in bed position, the lateral wings (21) are of collapsible quality. The angles of the lateral wings (21), in order to hinder the patient from falling off the bed, are designed as to arrange in desired levels according to the consciousness level of the patient. Thus those lateral wings ensure that the unit can turn into a large bed after an opening and can get small enough to get through the doors. The equipments, on which there are manually controlled equipments and on which the patient put his/her arms when the patient uses the unit as a chair, are demounted on the lateral wings (21). When the unit turns into a bed, the pillow (24) support at the head side is curved in an angle that will provide the pillow need of the patient. When a direction is given in order to turn the unit into chair position, the unit by curving its lateral wings (21) inside again, turns into a mobile chair which has enough sizes to get through the doors. That situation gains the patient the freedom of sleeping comfortably in standard bed sizes in any place. The lateral wings (21), by opening in wished angles with mechanic movement, provide the quality of conversion to bed in standard bed sizes.

### Provision of Physical Movement:

The support unit for conscious or unconscious physically handicapped patients which is designed for making the patient do physical movements characterized in that; it aims to hinder the constitution of bedsores on the body of the patient by fastening the blood circle of the patient through erecting the patient in different slants.

It ensures that the legs of the patient are curved from the knees and thus the legs move by keeping the patient in different slants and in upright position (25), by mobilizing the step (20) under the patients' feet up and down. There are safety belts (26) settled on the knee and chest which limits the unwelcome movements and hinders the patient from falling from the bed while the physical movements are done. The safety belts (26) support the patient to do movements in upright or other slants. The safety belt (26) that is attached to the knees of the patient is related to the step (20) on which the legs of the patient steps. The safety belt (26) and the step (20) do the movements together. The safety belt (26), designed differently for the knees, ensures that the knees of the patient are curved by moving with the step (20) simultaneously when the step moves upwards and by relaxing simultaneously ensures that the knees of the patient turn back to its former straight position when the step (20) moves downwards. The amount of pressure, which is made to the parts of the bed on which the patient touches, is proportionate with the slant of the body of the patient in vertical position (27). Thus the amount of pressure lessens when the patient gets closer to the upright position (25) from vertical position (27) and thanks to the movement the blood circle of the body gets normal. Thus the negative results like bed sores which are resulted from the pressure on the body are hindered through supporting the blood circle of the patient.

### Massaging under the Foot:

The support for the physically handicapped which is designed for unconscious physically handicapped patients characterized in that; it ensures that it fastens the blood circle of the patient by massaging under his/her feet in various angles and it relaxes the muscles of the patient. The step, on which the patient steps his/her feet by creating vibration, through the electro mechanic equipment it has, massages under the feet of the patient and ensures that the patient relaxes.

### BRIEF EXPLANATIONS OF THE FIGURES:

**FIGURE 1****:** The back view of the inner surface of the multi-functional pant (17) in which the inner surface is fixed on the body of the patient and the surroundings of the anus.
FIGURE 2: Isometric sectional view of the multi-functional pant.
FIGURE 3: Upper isometric sectional view of the multi-functional pant (17) fixed on the patient.
FIGURE 4: Sidelong general sectional view of the multi-functional pant (17) fixed on the patient.
FIGURE 5: Sidelong detailed sectional view of the waste removal electro mechanic pump (5).
FIGURE 6: Isometric sectional view of Latch (14).
FIGURE 7: Upper view of Latch (14).

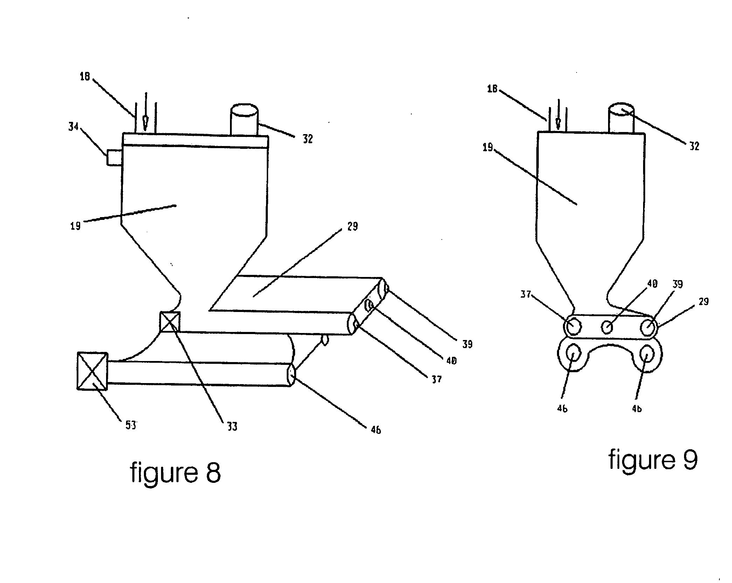

FIGURE 8: Isometric general sectional view of the waste reserve storage.
FIGURE 9: Front view of the waste reserve storage.

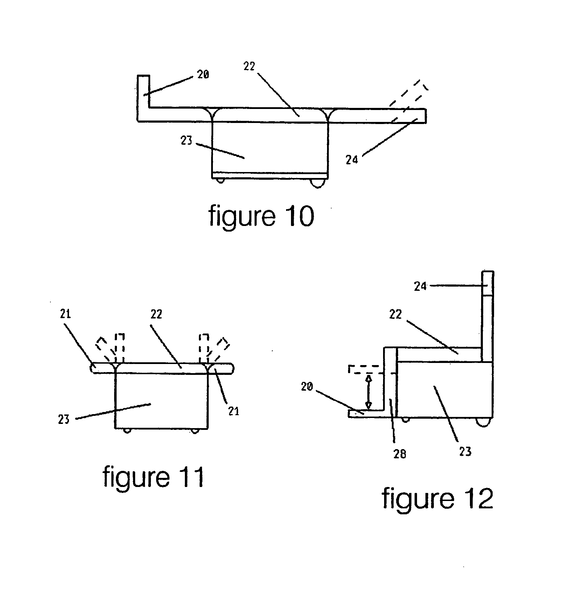

**FIGURE 10****:** Sidelong view of the unit in bed position.
FIGURE 11: The front view of the collapsible lateral wings of the unit.
**FIGURE 12****:** The sidelong view of the up and down movement of the step where the unit is in the chair position.

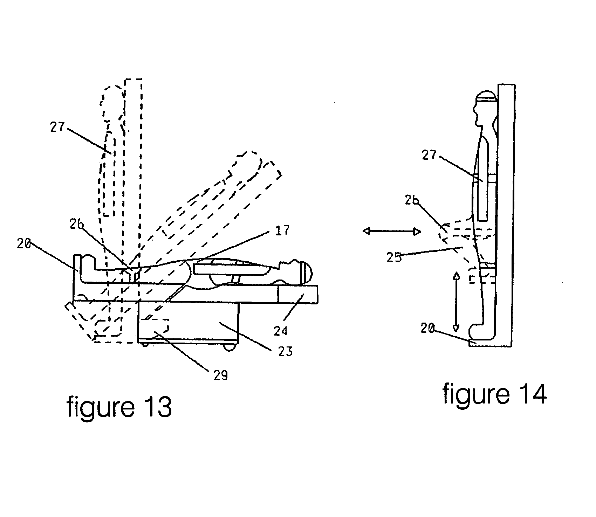

**FIGURE 13****:** The sidelong view of the different angled positions of the patient fixed on the bed.
**FIGURE 14**: The sidelong view of the physical movement position of the patient in vertical position.

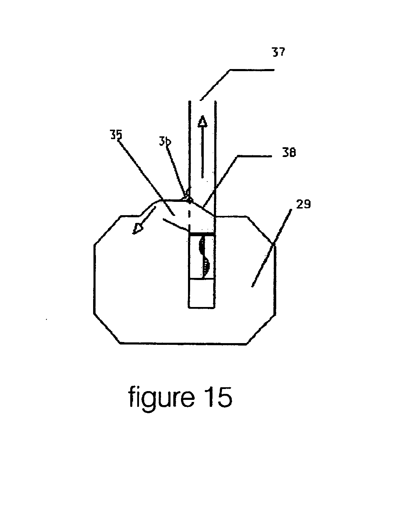

**FIGURE 15**: The upper sectional view of the bypass contrivance of the waste reserve storage.
FIGURE 16: The sidelong isometric view of the waste transfer station.
FIGURE 17: The front isometric view of the waste transfer station.
FIGURE 18: The sidelong isometric view fixed on the unit profile of the waste transfer station fixed on the closet.

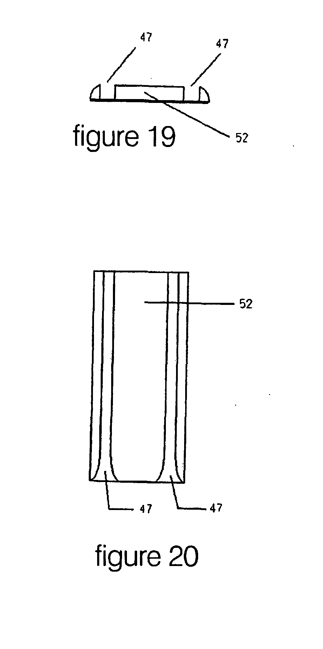

FIGURE 19: The front isometric view of the unit alignment panel.
FIGURE 20: The upper view of the unit alignment panel.

### THE EXPLANATIONS OF THE PARTS THAT ARE CONSISTENT IN THE UNIT FOR THE PHYSICALLY HANDICAPPED PATIENTS;

1. The inner surface level that touches on the patient's body by dividing the gluteus
2. The hole surrounding the anus of the patient
3. The level having multi-divisional cabins
4. Clean water division that will be used for the mobile multi-functional pant
5. Waste removal electro mechanic pump
6. The levels constituting distances of enough length
7. The strait which is the waste entry part of the waste removal pump (5)
8. The point of the waste removal pump (5) that touches the surroundings of the anus
9. Micro camera fixation channel
10. Waste control sensor fixation channel
11. Air entry fixation channel
12. Water entry fixation channel
13. Light source fixation channel
14. The latch
15. Electro mechanism operating the latch
16. Pneumatic diaphragm
17. Multi-functional pant
18. The pipe transferring the wastes of the multi functional pant
19. Waste reserve storage
20. The step of the unit moving up and down
21. The collapsible lateral wings of the unit
22. The seat section of the unit
23. The cabin of the unit
24. The pillow support of the unit
25. Upright position
26. The mobile knee-guard belt of the patient
27. Vertical position
28. Mobile step chassis
29. The waste transfer mechanism of the unit
30. The waste transfer station
31. The waste transfer electro mechanically equipped pump engine
32. Gas filter
33. The waste transfer pump
34. The waste level sensor
35. The waste inner circulation channel
36. The mechanism operating the clamp
37. The waste transfer pipe
38. The waste direction clamp
39. Clean water pipe
40. DC energy transfer battery
41. The sprinkler washing the parts of the unit through which the wastes go to the sewage system
42. The closet
43. The closet fixation screws
44. The station waste pipe part
45. The sewage system pipe
46. The shaft providing the back and forth movement of the waste reserve storage
47. The alignment of the unit to the waste transfer station channels
48. The current electronic control centre in the mobile-multifunctional pant
49. The waste reserve storage of the mobile multi-functional pant
50. The clean water storage of the multi-functional storage
51. DC battery cabin of the multi-functional pant
52. The unit alignment panel
53. Waste transfer mechanism electro mechanic equipped engine with reducer
54. Pump (5) strait clasp

## Claims

1. Support unit, comprising:
a multi-functional pant (17) which comprises:
- a first inner surface level (1) adapted to touch the patient's body;
- a second outer surface level (3);
- a hole (2) that is in the middle of the first inner surface level (1) and is adapted to be aligned with the middle of the anus, surrounding it;
- a micro camera fixation channel (9);
- a waste control sensor fixation channel (10);
- an air entry fixation channel (11); and
- and a water entry fixation channel (12);
**characterized in that** the multi-functional pant (17) further comprises:
- an electro mechanical waste removal pump (5) having a strait part (7), that is the waste entry part, wherein said hole (2) is adapted to centre said electro mechanical waste removal pump (5) and, depended on that, the strait part (7) around the anus;
- a light, that is adapted to enlighten the surroundings of the anus; and
- a fixation channel (13) on the outer edge of the waste entry strait part (7) of the electro mechanic waste removal pump (5);
wherein the multi-functional pant (17) further comprises cabins divided into levels (6) constituting long enough distances between the outer surface of the first inner surface level (1) and the innen surface (4) of the second outer surface level (3), for protecting the electromechanically equipped waste removal pump (5) from the pressure that the patient makes on said multi-functional pant (17).

2. Support unit according to claim 1, **characterized by** further comprising: a waste transfer mechanism (29) which has a pipe (18), settled in the cabin (23) of the unit under the patient, adapted to remove the wastes from the patient, a waste reserve storage (19) where the wastes are collected, under that storage waste transfer pump (33) fixed on the storage mixing the wastes and transferring to the station, alignment channels (47) performing the clamping and separating situation with the waste transfer station, gas filter (32) for stocked waste gases, waste level sensor (34), waste transfer pipe (37) where the wastes are pumped, mechanism (36) operating the valve which is fixed through by passing front of it, waste direction valve (38) which is on the tip of the part in the place where the waste transfer pipe (37) divided into two is combined, controlled by the unit, which has mechanical movements, will close one of the holes of the pipes on its both sides; and
a waste transfer station (30) which, by providing the connection of the unit with the sewage system, is adapted to transfer the wastes, that are transferred from the unit, to the sewage system, has two solenoids one of which is adapted to transfer water to the clean water pipe (39) attached to the city water, and one of which is adapted to transfer water to the sprinkler (41) that washes the parts of the station where the wastes go to the sewage system, which has a station waste pipe part (44) that is fixed, instead of the closet (42), on the sewage system pipe (45) on the ground.

3. The support unit according to claim 1 or 2, **characterized in that** the distance that is constituted between the outer surface of the first inner level (1) adapted to touch the body and the inner surface (4) of the second outer level (3) is for creating cabins in order to protect the electro mechanically equipped waste removal pump (5) from the pressure that the patient makes on the pant.

4. The support unit according to claim 1, **characterized in that** the waste entry strait (7) of the electromechanically equipped waste removal pump (5) is adapted to get close to the anus by being steered through the hole (2) that is in the middle of the first inner surface level (1), that touches the body of the patient, and that aligns with the middle of the anus.

5. The support unit according to claim 1 or 2, **characterized in that** the waste removal pump (5) is adapted to remove the wastes that comes out of the anus through waste entry strait (7) without touch on the body of the patient.

6. The support unit according to claim 1, **characterized in that** the strait part (7) which is adapted to surround the anus, being close to anus but not touching the anus, is of cylindrical shape that has a length of the opening diameter of the anus.

7. The support unit according to claim 1, **characterized in that** it includes a latch (14) that is adapted to close the space between the waste entry strait (7) of the electro mechanic waste removal pump (5) and the hole (2) surrounding the anus, that is adapted to cut the connection between the waste removal pump (5), the storage in the unit and the anus by closing the waste entry strait (7) between the anus and the pump after the removal process finishes, that moves through clasp mechanism or electro mechanism (15).

8. The support unit according to claim 1, **characterized in that** it includes a pneumatic diaphragm (16) that swells with the air consistent under the waste removal pump (5) after the wastes leave the body, that hinders the leak of wastes outside by ensuring that the point (8) of the upper waste entry strait (7) part of the pump that is adapted to touche the anus touches on the surroundings of the anus (2) with proper pressure through raising the waste removal pump (5) up.

9. The support unit according to claim 1, **characterized in that** the inner surface (1) of the first inner level that is adapted to touche the patient's body is covered with flexible material and cloth that does not affect blood circulation.

10. The support unit according to claim 1, **characterized in that** the pneumatic diaphragm (16), the waste removal pump (5), the point (8) of entry strait, when the patient is in bed position, by touching on the surroundings of the anus automatically, hinder the leak of the involuntary liquids etc. that may happen in the anus to the unwanted regions

11. The support unit according to claim 2, **characterized in that** it is adapted to bring the patient to the chair position through the warning of the waste control sensor (10) consistent in the multifunctional pant (17) as soon as the wastes leave the body while the patient is defecating in whatever position he/she is, and it is adapted to bring the patient to the squatting position through raising up of the moving step and its moving chassis (28), to which the step (20) under the patient's feet is attached, with the help of the mechanism.

12. The support unit according to claim 2, **characterized in that** it is adapted, when wished, to turn into a bed of one-person standard bed size, or it is adapted, when wished, to turn into a chair, is of collapsible quality when the patient is in bed position in the unit, which has arrangible sizes in wished sizes, the lateral wings (21) on which the patient puts his/her arms and which is demounted on the manual control equipment, a pillow support (24) on its head part which can curve in an angle that will fulfill the needs of the patient.

13. The support unit according to claim 2, **characterized in that** it is adapted to place the patient's legs bent from the knees, so that the patient is able to be moved through keeping the patient attached to the unit in the different slants, in the upright position (25), and by moving up and down the step (20) under the patient's feet through mechanical movements.

14. The support unit according to claim 2, **characterized in that** it further comprises safety belts (26), placed at the knee and chest region, that hinders the patient from falling down the bed side, the safety belt (26) attached to the knees is related with the step (20) under the patient's feet, and the safety belt (26), attached on the knees, by moving proportionally with the step (20) when the step (20) moves up and down and thus by curving the patient's knees and turning the patient's knees into their former straight position by removing back when the step (20) moves down ensures that the patient does physical movements.

15. The support unit according to claim 1, **characterized in that** the step (20), on which the patient steps, is adapted to give a massage under the lower base of the feet of the patients who have lost the ability of physical movement, by the vibration, thanks to the electromechanical equipment it has.

16. The support unit according to claim 1, **characterized in that** the aim of the distance that is constituted between the outer surface of the first inner level (1) and the inner surface of the second outer level (3) is to create cabin in order to save the electromechanically equipped waste removal pump (5) from the pressure that is made on the pant by the patient and is to create cabin for the waste reserve storage (49), clean water entry (50), DC energy battery (51).

17. The support unit according to claim 1, **characterized in that** the first inner level (1) of the part that is adapted to touch the patient of the body is covered with flexible material and cloth that is non-sweating and antibacterial.

18. The support unit according to claim 1, **characterized in that** an electromechanically equipped waste removal pump (5), by removing the pump to the waste storage (49), is adapted to give a warning alert through the level sensor consistent in it when the wastes fill the storage.

19. The support unit according to claim 1, **characterized in that** the mobile multi-functional pant, in order to remove the wastes in the waste reserve storage (49), is adapted to attach the waste reserve storage to the unit so that the wastes go through the waste removal mechanism (29).

## Patentansprüche

1. Hilfeeinheit mit:
einer Multifunktions-Hose (17), welche umfasst:
- eine erste Innenflächenebene (1), die dazu ausgebildet ist, den Körper des Patienten zu berühren;
- eine zweite Außenflächenebene (3);
- ein Loch (2), das sich in der Mitte der ersten Innenflächenebene (1) befindet und dazu ausgebildet ist, mit der Mitte des Anus ausgerichtet zu sein, den es umgibt;
- einen Mikrokamera-Befestigungskanal (9);
- einen Ausscheidungsprodukt-Steuersensor-Befestigungs-kanal (10);
- einen Lufteintritt-Befestigungskanal (11); und
- und einen Wassereintritt-Befestigungskanal (12);
**dadurch gekennzeichnet, dass** die Multifunktions-Hose (17) ferner umfasst:
- eine elektromechanische Ausscheidungsprodukt-Entfernungspumpe (5) mit einem Engenteil (7), welches das Ausscheidungsprodukt-Eintrittsteil darstellt, wobei das Loch (2) dazu ausgebildet ist, die elektromechanische Ausscheidungsprodukt-Entfernungspumpe (5) und, in Abhängigkeit von diesem, das Engenteil (7) rund um den Anus zu zentrieren;
- eine Lampe, die dazu ausgebildet ist, die Umgebung des Anus zu beleuchten; und
- einen Befestigungskanal (13) auf der äußeren Kante des Ausscheidungsprodukteintritts-Engenteils (7) der elektromechanischen Ausscheidungsprodukt-Entfernungspumpe (5);
wobei die Multifunktions-Hose (17) ferner Abteilungen umfasst, die in Ebenen (6) unterteilt sind, welche ausreichend lange Abstände zwischen der Außenfläche der ersten Innenflächenebene (1) und der Innenfläche (4) der zweiten Außenflächenebene (3) zum Schützen der elektromechanisch ausgestatteten Ausscheidungsprodukt-Entfernungspumpe (5) vor dem Druck bilden, den der Patient auf die Multifunktions-Hose (17) ausübt.

2. Hilfeeinheit nach Anspruch 1, **dadurch gekennzeichnet, dass** sie ferner umfasst:
einen Ausscheidungsprodukt-Transportmechanismus (29), der ein Rohr (18) aufweist, das in der Abteilung (23) der Einheit unter dem Patienten eingerichtet und dazu ausgebildet ist, die Ausscheidungsprodukte von dem Patienten zu entfernen, einen Ausscheidungsprodukt-Reservespeicher (19), in welchem die Ausscheidungsprodukte gesammelt werden, unter jener Speicherausscheidungsprodukt-Transportpumpe (33), die an dem Speicher befestigt ist, der die Ausscheidungsprodukte vermischt und zur Station transportiert, Ausrichtungskanäle (47), die die Einklemm- und Trennsituation mit der Ausscheidungsprodukt-Transportstation durchführen, ein Gasfilter (32) für gelagerte Ausscheidungsproduktgase, einen Ausscheidungsproduktpegelsensor (34), ein Ausscheidungsprodukt-Transportrohr (37), in welchem die Ausscheidungsprodukte gepumpt werden, einen Mechanismus (36), der das Ventil betätigt, welches durchgehend befestigt ist, indem es vor diesem vorbeigeführt ist, ein Ausscheidungsprodukt-Richtungsventil (38), das sich an der Spitze des Teils an dem Ort befindet, an dem das zweigeteilte Ausscheidungsprodukt-Transportrohr (37) kombiniert ist, und von der Einheit gesteuert wird, die mechanische Bewegungen aufweist, und eines der Löcher der Rohre auf seinen beiden Seiten schließt; und
eine Ausscheidungsprodukt-Transportstation (30), die durch Bereitstellen der Verbindung der Einheit mit dem Abwassersystem dazu ausgebildet ist, die Ausscheidungsprodukte zu transportieren, die von der Einheit zum Abwassersystem transportiert werden, die zwei Elektromagnete aufweist, von denen einer dazu ausgebildet ist, Wasser zum Reinwasserrohr (39) zu transportieren, das an die öffentliche Wasserleitung angeschlossen ist, und von denen einer dazu ausgebildet ist, Wasser zu der Sprinkleranlage (41) zu transportieren, die die Teile der Station wäscht, an der die Ausscheidungsprodukte in das Abwassersystem gelangen, die ein Stations-Ausscheidungsprodukt-Rohrteil (44) aufweist, das anstelle an der Toilette (42) an dem Abwassersystemrohr (45) auf dem Boden befestigt ist.

3. Hilfeeinheit nach Anspruch 1 oder 2, **dadurch gekennzeichnet, dass** der Abstand, der zwischen der Außenfläche der ersten Innenebene (1), die dazu ausgebildet ist, den Körper zu berühren, und der Innenfläche (4) der zweiten Außenebene (3) gebildet ist, zum Erzeugen von Abteilungen dient, um die elektromechanisch ausgestattete Ausscheidungsprodukt-Entfernungspumpe (5) gegen den Druck zu schützen, den der Patient auf die Hose ausübt.

4. Hilfeeinheit nach Anspruch 1, **dadurch gekennzeichnet, dass** die Ausscheidungsprodukt-Eintrittsenge (7) der elektromechanisch ausgestatteten Ausscheidungsprodukt-Entfernungspumpe (5) dazu ausgebildet ist, nahe an den Anus zu gelangen, indem sie durch das Loch (2) gesteuert wird, das sich in der Mitte der ersten Innenflächenebene (1) befindet, welches den Körper des Patienten berührt und das mit der Mitte des Anus ausgerichtet ist.

5. Hilfeeinheit nach Anspruch 1 oder 2, **dadurch gekennzeichnet, dass** die Ausscheidungsprodukt-Entfernungspumpe dazu ausgebildet ist, die Ausscheidungsprodukte zu entfernen, die aus dem Anus durch die Ausscheidungsprodukt-Eintrittsenge (7) kommen, ohne den Körper des Patienten zu berühren.

6. Hilfeeinheit nach Anspruch 1, **dadurch gekennzeichnet, dass** das Engenteil (7), das dazu ausgebildet ist, den Anus zu umgeben, wobei es sich nahe am Anus befindet, aber den Anus nicht berührt, von zylindrischer Form ist, die eine Länge des Öffnungsdurchmessers des Anus aufweist.

7. Hilfeeinheit nach Anspruch 1, **dadurch gekennzeichnet, dass** sie einen Verschluss (14) beinhaltet, der dazu ausgebildet ist, den Raum zwischen der Ausscheidungsprodukt-Eintrittsenge (7) der elektromechanischen Ausscheidungsprodukt-Entfernungspumpe (5) und dem den Anus umgebenden Loch (2) zu verschließen, der dazu ausgebildet ist, die Verbindung zwischen der Ausscheidungsprodukt-Entfernungspumpe (5), dem Speicher in der Einheit und dem Anus durch Schließen der Ausscheidungsprodukt-Eintrittsenge (7) zwischen dem Anus und der Pumpe nach dem Beenden des Entfernungsvorgangs zu unterbrechen, die sich durch einen Schließmechanismus oder Elektromechanismus (15) bewegt.

8. Hilfeeinheit nach Anspruch 1, **dadurch gekennzeichnet, dass** sie eine pneumatische Membran (16) einschließt, die mit der unter der Ausscheidungsprodukt-Entfernungspumpe (5) vorhandenen Luft anschwillt, nachdem die Ausscheidungsprodukte den Körper verlassen haben, die das Austreten von Ausscheidungsprodukten nach außen verhindert, indem sie sicherstellt, dass der Punkt (8) des oberen Teils der Ausscheidungsprodukt-Eintrittsenge (7) der Pumpe, das dazu ausgebildet ist, den Anus zu berühren, die Umgebung des Anus (2) mit entsprechendem Druck durch Höherstellen der Ausscheidungsprodukt-Entfernungspumpe (5) berührt.

9. Hilfeeinheit nach Anspruch 1, **dadurch gekennzeichnet, dass** die Innenfläche (1) der ersten inneren Ebene, die dazu ausgebildet ist, den Körper des Patienten zu berühren, mit flexiblem Material und Tuch abgedeckt ist, das den Blutkreislauf nicht beeinträchtigt.

10. Hilfeeinheit nach Anspruch 1, **dadurch gekennzeichnet, dass**, wenn sich der Patient in der Bettposition befindet, die pneumatische Membran (16), die Ausscheidungsprodukt-Entfernungspumpe (5) und der Punkt (8) der Eintrittsenge das Austreten der unwillkürlichen Flüssigkeiten usw., die im Anus entstehen, in die ungewollten Bereiche durch Berühren der Umgebung des Anus automatisch verhindern.

11. Hilfeeinheit nach Anspruch 2, **dadurch gekennzeichnet, dass** sie dazu ausgebildet ist, den Patienten durch das Warnen des Ausscheidungsprodukt-Steuersensors (10), der in der Multifunktions-Hose (17) vorhanden ist, in die Stuhlposition zu bringen, sobald die Ausscheidungsprodukte den Körper verlassen, während der Patient in jeglicher Position, der er sich befindet, defäkiert, und sie dazu ausgebildet ist, den Patienten durch das Anheben der Bewegungsstufe und ihres Bewegungschassis (28), an welchem die Stufe (20) unter den Füßen des Patienten angebracht ist, mit der Hilfe des Mechanismus in die hockende Position zu bringen.

12. Hilfeeinheit nach Anspruch 2, **dadurch gekennzeichnet, dass** sie dazu ausgebildet ist, sich, wenn gewünscht, in ein Bett einer Ein-Personen-Standardbettgröße zu verwandeln, oder dass sie dazu ausgebildet ist, sich, wenn gewünscht, in einen Stuhl zu verwandeln, dass sie von zusammenklappbarer Qualität ist, wenn sich der Patient in der Einheit in der Bettposition befindet, welche arrangierbare Größen in gewünschten Größen, die seitlichen Flügel (21), auf welchen der Patient seine Arme ablegt und die an der manuellen Steuerausstattung abmontiert sind, und eine Kissenstütze (24) an seinem Kopfteil aufweist, die sich in einer Winkel krümmen kann, der die Bedürfnisse des Patienten erfüllt.

13. Hilfeeinheit nach Anspruch 2, **dadurch gekennzeichnet, dass** sie dazu ausgebildet ist, die vom Knie gebeugten Beine des Patienten zu platzieren, so dass der Patient durch weiteres Halten des Patienten in den unterschiedlichen Neigungen an der Einheit, in der aufrechten Position (25) und durch Auf- und Abbewegen der Stufe (20) unter den Füßen des Patienten durch mechanische Bewegungen bewegt werden kann.

14. Hilfeeinheit nach Anspruch 2, **dadurch gekennzeichnet, dass** sie ferner Sicherheitsgurte (26) umfasst, die im Knie- und Brustbereich platziert sind und den Patienten daran hindern, an der Bettseite hinunterzufallen, wobei der an den Knien angebrachte Sicherheitsgurt (26) mit der Stufe (20) unter den Füßen des Patienten zusammenhängt und der an den Knien angebrachte Sicherheitsgurt (26), indem er sich proportional mit der Stufe (20) bewegt, wenn sich die Stufe (20) auf und ab bewegt, und somit durch Beugen der Knie des Patienten und Drehen der Knie des Patienten in ihrer frühere gerade Position durch Zurückbewegen, wenn sich die Stufe (20) abwärts bewegt, sicherstellt, dass der Patient Körperbewegungen ausführt.

15. Hilfeeinheit nach Anspruch 1, **dadurch gekennzeichnet, dass** die Stufe (20), auf welche der Patient tritt, dazu ausgebildet ist, eine Massage unter der Unterseite der Füße der Patienten, die die Fähigkeit zur Körperbewegung verloren haben, durch die Vibration aufgrund der elektromechanischen Ausstattung, über die sie verfügt, zu verabreichen.

16. Hilfeeinheit nach Anspruch 1, **dadurch gekennzeichnet, dass** das Ziel des Abstands, der zwischen der Außenfläche des ersten Innenebene (1) und der Innenfläche der zweiten Außenebene (3) gebildet ist, darin besteht, eine Abteilung zu erzeugen, um die elektromechanisch ausgestattete Ausscheidungsprodukt-Entfernungspumpe (5) gegen den Druck zu schützen, der durch den Patienten auf die Hose ausgeübt wird, und eine Abteilung für den Ausscheidungsprodukt-Reservespeicher (49), den Reinwassereintritt (50) und die Gleichstrom-Energiebatterie (51) zu schaffen.

17. Hilfeeinheit nach Anspruch 1, **dadurch gekennzeichnet, dass** die erste Innenebene (1) des Teils, das dazu ausgebildet ist, den Patienten des Körpers zu berühren, mit elastischem Material und Tuch bedeckt ist, das transpirationsfrei und antibakteriell ist.

18. Hilfeeinheit nach Anspruch 1, **dadurch gekennzeichnet, dass** eine elektromechanisch ausgestattete Ausscheidungsprodukt-Entfernungspumpe (5) durch Entfernen der Pumpe zum Ausscheidungsproduktspeicher (49) dazu ausgebildet ist, eine Warnung durch den Pegelsensor auszugeben, der in diesem vorhanden ist, wenn die Ausscheidungsprodukte den Speicher füllen.

19. Hilfeeinheit nach Anspruch 1, **dadurch gekennzeichnet, dass** die mobile Multifunktions-Hose dazu ausgebildet ist, zum Entfernen der Ausscheidungsprodukte im Ausscheidungsprodukt-Reservespeicher (49) den Ausscheidungsprodukt-Reservespeicher an der Einheit so zu befestigen, dass die Ausscheidungsprodukte durch den Ausscheidungsprodukt-Entfernungsmechanismus (29) gehen.

## Revendications

1. Unité de support, comprenant :
une culotte multifonctionnelle (17) qui comprend :
- un premier niveau de surface intérieure (1) adapté à toucher le corps du patient ;
- un deuxième niveau de surface extérieure (3) ;
- un trou (2) qui est dans le milieu du premier niveau de surface intérieure (1) et est adapté à être aligné avec le milieu de l'anus, entourant celui-ci ;
- un canal de fixation (9) de microcaméra ;
- un canal de fixation (10) de capteur de contrôle de déchets ;
- un canal de fixation (11) d'entrée d'air ; et
- un canal de fixation (12) d'entrée d'eau ;
**caractérisée en ce que** la culotte multifonctionnelle (17) comprend en outre :
- une pompe (5) électromécanique d'élimination de déchets ayant une partie rectiligne (7), qui est la partie d'entrée de déchets, dans laquelle ledit trou (2) est adapté à centrer ladite pompe (5) électromécanique d'élimination de déchets et, dépendant de celle-ci, la partie rectiligne (7) autour de l'anus ;
- une lumière, qui est adaptée à éclairer les alentours de l'anus ; et
- un canal de fixation (13) sur le bord extérieur de la partie rectiligne (7) d'entrée de déchets de la pompe (5) électromécanique d'élimination de déchets ;
dans laquelle la culotte multifonctionnelle (17) comprend en outre des chambres divisées en niveaux (6) constituant des distances suffisamment longues entre la surface extérieure du premier niveau de surface intérieure (1) et la surface intérieure (4) du deuxième niveau de surface extérieure (3), pour protéger la pompe (5) électromécanique d'élimination de déchets de la pression que le patient exerce sur ladite culotte multifonctionnelle (17).

2. Unité de support selon la revendication 1, **caractérisée en ce que** comprenant en outre :
un mécanisme (29) de transfert de déchets qui a un tuyau (18), logé dans la chambre (23) de l'unité sous le patient, adapté à éliminer les déchets du patient, un stockage (19) de réserve de déchets où les déchets sont collectés, sous cela une pompe (33) de transfert de déchets de stockage fixée sur le stockage mélangeant les déchets et
les transférant jusqu'à la station, des canaux (47) d'alignement exécutant le situation de fixation et de séparation avec la station de transfert de déchets, un filtre à gaz (32) pour des déchets gazeux stockés, un capteur (34) de niveau de déchets, un tuyau (37) de transfert de déchets où les déchets sont pompés, un mécanisme (36) faisant fonctionner la soupape qui est fixée à travers en passant devant celle-ci, une soupape (38) d'orientation de déchets qui est sur la pointe de la partie à l'endroit où le tuyau (37) de transfert de déchets divisé en deux est combiné, commandée par l'unité, qui a des mouvements mécaniques, fermera un des trous des tuyaux sur ses deux côtés ; et
une station (30) de transfert de déchets qui, en prévoyant la connexion de l'unité avec le système d'égout, est adaptée à transférer les déchets, qui sont transférés hors de l'unité, jusqu'au système d'égout, a deux solénoïdes, l'un desquels est adapté à transférer de l'eau jusqu'au tuyau (39) d'eau propre relié à l'eau du réseau, et l'un desquels est adapté à transférer de l'eau jusqu'à l'arroseur (41) qui
lave les parties de la station où les déchets vont jusqu'au système d'égout, qui a une partie (44) de tuyau de déchets de station qui est fixe, à la place du cabinet (42), sur le tuyau (45) de système d'égout au sol.

3. Unité de support selon la revendication 1 ou 2, **caractérisée en ce que** la distance qui est constituée entre la surface extérieure du premier niveau intérieur (1) adapté à toucher le corps et la surface intérieure (4) du deuxième niveau extérieur (3) sert à créer des chambres afin de protéger la pompe (5) électromécanique d'élimination de déchets de la pression que le patient exerce sur la culotte.

4. Unité de support selon la revendication 1, **caractérisée en ce que** la partie rectiligne (7) d'entrée de déchets de la pompe (5) électromécanique d'élimination de déchets est adaptée à se rapprocher de l'anus en étant pilotée à travers le trou (2) qui est dans le milieu du premier niveau de surface intérieure (1), qui touche le corps du patient, et qui s'aligne sur le milieu de l'anus.

5. Unité de support selon la revendication 1 ou 2, **caractérisée en ce que** la pompe (5) d'élimination de déchets est adaptée à éliminer les déchets qui sortent de l'anus à travers la partie rectiligne (7) d'entrée de déchets sans toucher le corps du patient.

6. Unité de support selon la revendication 1, **caractérisée en ce que** la partie rectiligne (7) qui est adaptée à entourer l'anus, étant proche de l'anus mais ne touchant pas l'anus, est d'une forme cylindrique qui a une longueur du diamètre d'ouverture de l'anus.

7. Unité de support selon la revendication 1, **caractérisée en ce qu'**elle inclut un verrou (14) qui est adapté à fermer l'espace entre la partie rectiligne (7) d'entrée de déchets de la pompe (5) électromécanique d'élimination de déchets et le trou (2) entourant l'anus, qui est adapté à couper la connexion entre la pompe (5) d'élimination de déchets, le stockage dans l'unité et l'anus en fermant la partie rectiligne (7) d'entrée de déchets entre l'anus et la pompe lorsque le processus d'élimination est terminé, qui se déplace par l'intermédiaire d'un mécanisme de crochet ou d'un mécanisme électrique (15).

8. Unité de support selon la revendication 1, **caractérisée en ce qu'**elle inclut un diaphragme (16) pneumatique qui gonfle avec l'air existant sous la pompe (5) d'élimination de déchets après que les déchets ont quitté le corps, qui empêche la fuite de déchets à l'extérieur en assurant que le point (8) de la partie rectiligne (7) supérieure d'entrée de déchets de la pompe qui est adapté à toucher l'anus touche aux environs de l'anus (2) avec une pression appropriée par levage de la pompe (5) d'élimination de déchets.

9. Unité de support selon la revendication 1, **caractérisée en ce que** la surface intérieure (1) du premier niveau intérieur qui est adaptée à toucher le corps du patient est recouverte avec une matière souple et une étoffe qui n'a pas d'effet sur la circulation sanguine.

10. Unité de support selon la revendication 1, **caractérisée en ce que** le diaphragme (16) pneumatique, la pompe (5) d'élimination de déchets, le point (8) de partie rectiligne d'entrée, lorsque le patient est dans la position de lit, en touchant les environs de l'anus automatiquement, empêchent la fuite des liquides involontaires, etc., qui peut avoir lieu dans l'anus jusqu'aux régions indésirables.

11. Unité de support selon la revendication 2, **caractérisée en ce qu'**elle est adaptée à amener le patient en position de chaise par l'intermédiaire de l'avertissement du capteur (10) de contrôle de déchets existant dans la culotte multifonctionnelle (17) dès que les déchets sortent du corps tandis que le patient défèque quelle que soit la position dans laquelle il est, et qu'elle est adaptée à amener le patient en position d'accroupissement par élévation de la marche mobile et de son châssis (28) mobile, auquel la marche (20) sous les pieds du patient est fixée, à l'aide du mécanisme.

12. Unité de support selon la revendication 2, **caractérisée en ce qu'**elle est adaptée, lorsque tel est désiré, à se transformer en un lit de taille standard d'une personne, ou qu'elle est adaptée, lorsque tel est désiré, à se transformer en une chaise, est de qualité repliable lorsque le patient est dans la position de lit dans l'unité, qui a des tailles ajustables à des tailles désirées, les ailes latérales (21) sur lesquelles le patient pose ses bras et qui est démontée sur l'équipement à commande manuelle, un oreiller support (24) sur sa partie de tête qui peut s'incurver selon un angle qui satisfera aux besoins du patient.

13. Unité de support selon la revendication 2, **caractérisée en ce qu'**elle est adaptée à placer les jambes du patient repliées à partir des genoux, de sorte que le patient est apte à être déplacé tout en maintenant le patient attaché à l'unité dans les différentes orientations, dans la position verticale (25), et en faisant monter et descendre la marche (20) sous les pieds du patient par l'intermédiaire de mouvements mécaniques.

14. Unité de support selon la revendication 2, **caractérisée en ce qu'**elle comprend en outre des ceintures de sécurité (26), placées au niveau de la région des genoux et de la poitrine, qui empêchent que le patient tombe du bord du lit, la ceinture de sécurité (26) fixée aux genoux est associée avec la marche (20) sous les pieds du patient, et la ceinture de sécurité (26), fixée aux genoux, en se déplaçant proportionnellement avec la marche (20) lorsque la marche (20) monte et descend et ainsi en pliant les genoux du patient et en remettant les genoux du patient dans leur position droite antérieure en se déplaçant vers l'arrière lorsque la marche (20) descend, assure que le patient effectue des mouvements physiques.

15. Unité de support selon la revendication 1, **caractérisée en ce que** la marche (20), sur laquelle le patient repose, est adaptée à pratiquer un massage sous la base inférieure des pieds des patients qui ont perdu la capacité de mouvement physique, par la vibration, grâce à l'équipement électromécanique dont elle est dotée.

16. Unité de support selon la revendication 1, **caractérisée en ce que** le but de la distance qui est constituée entre la surface extérieure du premier niveau intérieur (1) et la surface intérieure du deuxième niveau extérieur (3) est de créer une chambre afin de protéger la pompe (5) électromécanique d'élimination de déchets de la pression qui est exercée sur la culotte par le patient et de créer une chambre pour le stockage (49) de réserve de déchets, l'entrée (50) d'eau propre, la batterie (51) d'énergie CC.

17. Unité de support selon la revendication 1, **caractérisée en ce que** le premier niveau intérieur (1) de la partie qui est adaptée à toucher le corps du patient est recouvert d'une matière flexible et d'une étoffe qui est anti-transpirante et antibactérienne.

18. Unité de support selon la revendication 1, **caractérisée en ce qu'**une pompe (5) électromécanique d'élimination de déchets, en déplaçant la pompe jusqu'au stockage (49) de déchets, est adaptée à donner un signal d'alerte par l'intermédiaire du capteur de niveau existant dans celui-ci lorsque les déchets remplissent le stockage.

19. Unité de support selon la revendication 1, **caractérisée en ce que** la culotte multifonctionnelle mobile, pour éliminer les déchets dans le stockage (49) de réserve de déchets, est adaptée à fixer le stockage de réserve de déchets sur l'unité de façon à ce que les déchets passent jusqu'au mécanisme (29) d'élimination de déchets.
